# EUROPÄISCHE PATENTSCHRIFT

(11) **EP 4 312 755 B1**
(45) Veröffentlichungstag und Bekanntmachung des Hinweises auf die Patenterteilung: **30.07.2025**
(21) Anmeldenummer: 22723339.2
(22) Anmeldetag: 22.03.2022
(51) Int. Cl.: A61B 5/11, A47C 31/12, A61B 5/103, A61B 5/00, A61N 1/04, A61N 1/36, A63B 21/002

(54) **TRAININGSVORRICHTUNG ZUM DURCHFÜHREN EINES MUSKELTRAININGS FÜR EINEN MUSKEL**
TRAINING DEVICE FOR CARRYING OUT MUSCLE TRAINING FOR A MUSCLE
DISPOSITIF D'ENTRAÎNEMENT POUR LA RÉALISATION D'UN ENTRAÎNEMENT MUSCULAIRE POUR UN MUSCLE

(30) Priorität: 23.03.2021 DE 102021107223
(43) Veröffentlichungstag der Anmeldung: 07.02.2024
(73) Patentinhaber: Headis GmbH, 66919 Weselberg (DE)
(72) Erfinder: WEGNER, Rene, 66919 Weselberg (DE)
(74) Vertreter: Weidner Stern Jeschke
(86) Internationale Anmeldenummer: PCT/DE2022/200053
(87) Internationale Veröffentlichungsnummer: WO 2022/199766

(56) Entgegenhaltungen:
- DE-A1- 102015 002 565
- JP-A- 2014 068 659
- US-A1- 2007 208 392

## Beschreibung

Die Erfindung betrifft eine Trainingsvorrichtung zum Durchführen eines Muskeltrainings eines Muskels, insbesondere eines bestimmten Muskels, einer Person mit einer Steuereinheit, einem Grundkörper und einer am Grundkörper angeordneten Belastungsmesseinrichtung, wobei der Muskel, insbesondere der bestimmte Muskel, in einer Nutzungsposition der Person in Kontakt mit der Belastungsmesseinrichtung ist und die Belastungsmesseinrichtung einen Belastungssensor oder mehrere Belastungssensoren zum Bestimmen einer durch die Person auf die Belastungsmesseinrichtung ausgeübten Belastung an einem jeweiligen mit einem Teilbereich des Muskels korrelierten Messpunkt an einer jeweiligen Messpunktposition aufweist.

Trainingsvorrichtungen der genannten Art sind beispielsweise derart bekannt, dass eine Sitzposition einer auf einem Stuhl sitzenden Person mittels entsprechender Belastungssensoren detektierbar ist. Weiterhin sind Belastungssensoren in beispielsweise Sitzmöbeln oder anderen Einrichtungen grundsätzlich bekannt. Allerdings ist mit entsprechenden Vorrichtungen kein interaktives Training möglich. Das Dokument JP2014068659A offenbart eine Trainingsvorrichtung gemäß dem Oberbegriff des Anspruchs 1.

Aufgabe der Erfindung ist es, den Stand der Technik zu verbessern.

Gelöst wird die Aufgabe durch eine Trainingsvorrichtung gemäß den Merkmalen des Anspruchs 1 gelöst.

Damit kann im Zusammenwirken der Steuereinheit, der Signaleinheit und der Belastungsmesseinrichtung ein interaktives Agieren mit der Trainingsvorrichtung erfolgen, indem beispielsweise die Person mittels der Signaleinheit ein Signal zum Durchführen einer bestimmten Muskelkontraktion erhält und bei einem erfolgreichen Durchführen der Muskelkontraktion ein positives Erfolgssignal, beispielsweise eine positive Rückmeldung mittels der Signaleinheit, erhält und damit der Trainingserfolg direkt erkennbar und beispielsweise auch langfristig protokollierbar ist. Im umgekehrten Fall würde bei einem beispielsweise noch nicht ausreichenden Kontrahieren des entsprechenden Muskels ein negatives Erfolgssignal derart ausgegeben, dass beispielsweise eine Motivation zu einer weiteren Muskelkontraktion erfolgt.

Folgende Begriffe seien in diesem Zusammenhang erläutert:
Eine "Trainingsvorrichtung" beschreibt eine technische, insbesondere interaktive, Einrichtung, mittels der eine Person einen bestimmten Muskel trainieren kann, wobei die Trainingsvorrichtung beispielsweise mechanische Bauteile, Sensorik und eine entsprechende Steuereinheit aufweist und damit auch eine Rückmeldung an die Person geben kann. Das "Durchführen eines Muskeltrainings" beschreibt dabei das interaktive Kontrahieren eines Muskels, insbesondere eines bestimmten Muskels, womit beispielsweise eine therapeutische Wirkung für den Muskel, eine Kräftigung des Muskels oder eine langfristige Entspannung des Muskels durch eine entsprechend angeleitete und gesteuerte kurzfristige Kontraktion des entsprechenden Muskels erreicht wird. Ein "Muskel" ist dabei ein kontraktionsfähiges Organ eines Lebewesens und in diesem Zusammenhang nicht nur als einzelner Muskel, sondern auch weiter gefasst als Muskelgruppe, die beispielsweise repräsentativ für eine bestimmte Bewegung oder ein bestimmtes Trainingsziel ist, zu verstehen.

Ein "bestimmter Muskel" bezeichnet in diesem Zusammenhang, dass ein definierter, beispielsweise sportmedizinisch zu trainierender, Muskel oder analog dazu eine entsprechende Muskelgruppe abgegrenzt von beispielsweise umliegenden Muskeln oder auch umliegenden Muskelgruppen entsprechend mittels der Belastungssensoren detektiert und in der Folge gezielt trainiert werden kann, sodass beispielsweise im Rahmen eines Muskeltrainings, einer physiotherapeutischen Behandlung oder auch eine sportmedizinischen Diagnostik die entsprechende Muskelkontraktion dieses bestimmten Muskels durchgeführt und bewertet werden kann. So ist dieser bestimmte Muskel beispielsweise ein Flexor, also ein Beugemuskel, welcher beispielsweise an einer Rückseite eines Oberschenkels angeordnet ist. Eine "Person" ist beispielsweise ein Mensch, wobei die Trainingsvorrichtung nicht nur für Menschen, sondern auch für eine Vielzahl von Lebewesen mit entsprechenden Muskeln, also beispielsweise auch für ein entsprechendes Training von Tieren, verwendbar ist.

Eine "Steuereinheit" kann eine insbesondere elektronische Einheit und/oder ein Computer sein, welche dazu eingerichtet ist, entsprechende Signale aufzunehmen, mittels beispielsweise eines Algorithmus daraus entsprechende Ergebnisse zu erzeugen und diese auszugeben, sodass beispielsweise mittels einer Ausgabe entsprechende weitere technische Einheiten gesteuert, angeregt oder beeinflusst werden.

Ein "Grundkörper" ist beispielsweise ein Rahmen, ein Grundgestell oder ein anderes mechanisches Bauteil, welches wesentliche Bestandteile der Trainingsvorrichtung insbesondere in einem geometrischen Zusammenhang hält. So kann ein Grundkörper der Trainingsvorrichtung beispielsweise das Gestell eines Stuhles sein, jedoch kann der Grundkörper auch flexibel, biegeschlaff oder vergleichbar ausgestaltet sein und beispielsweise durch eine Gewebeschicht in Form einer Matte oder einer Sportunterlage gebildet sein.

Eine "Belastungsmesseinrichtung" kann eine technische, mechanische oder auch elektromechanische oder elektronische Einrichtung sein, mittels der eine Belastung aufgenommen und messtechnisch verarbeitet werden kann. Im einfachsten Falle ist eine solche Belastungsmesseinrichtung dabei eine mechanisch übersetzte Wäge-Einrichtung, wobei auch elektronische Ausgestaltungen zum Bestimmen und elektronischen Umwandeln von entsprechenden Belastungen damit bezeichnet sein können. Mittels dieser Belastungsmesseinrichtungen wird dann ein "Belastungsprofil" aufgenommen, wobei dieses Belastungsprofil dabei eine beispielsweise örtliche Verteilung unterschiedlicher Belastungen oder eine örtliche Veränderung unterschiedlicher Belastungen entlang einer Dimension, zweier Dimensionen oder auch dreier Raumdimensionen, insbesondere auch in Abhängigkeit von einem zeitlichen Verlauf, beschreibt. In diesem Zusammenhang sei darauf hingewiesen, dass die jeweiligen Begriffe im Zusammenhang mit der Bezeichnung "Belastung" dabei im einfachsten Falle eine Belastung im Sinne einer Kraft, eines Druckes, jedoch auch eine davon abgeleitete Größe wie beispielsweise eine Kontaktfeststellung oder eine Veränderungsgeschwindigkeit oder Veränderung eines Kontaktes, einer Kraft oder eines Druckes oder einer anderen Belastungsgröße bezeichnen kann, die jeweils aussagekräftig für die Muskelkontraktion ist oder sind.

Eine "Nutzungsposition" der Person ist dabei die Position, die die Person oder das Lebewesen zum zweckmäßigen Benutzer der Trainingsvorrichtung einnimmt. Auf einem Stuhl wäre diese Nutzungsposition beispielsweise das aufrechte Sitzen, auf einer Matte ein entsprechendes flaches Liegen. Für andere Trainingsvorrichtungen ist die Nutzungsposition analog dazu einzunehmen.

Ein "Kontakt" beschreibt den physischen Kontakt des jeweiligen Muskels mit der Belastungsmesseinrichtung an einem bestimmten Punkt oder beispielsweise über eine bestimmte Fläche, sodass beispielsweise die Person auf der Belastungsmesseinrichtung liegt, sitzt oder in anderer Form mit der Belastungsmesseinrichtung in Kontakt gebracht und/oder in Kontakt gehalten wird.

Die Belastungsmesseinrichtung weist dabei unterschiedliche "Belastungssensoren" oder auch nur einen Belastungssensor auf, welcher als mechanischer, elektromechanischer oder auch elektronischer Sensor ausgebildet sein kann und eine entsprechende "Belastung" gemäß obiger Beschreibung detektieren und beispielsweise in Form eines mechanischen oder auch elektronischen Signals weiterleiten kann. Dazu ist ein entsprechender "Teilbereich" des Muskels, also ein bestimmter Abschnitt eines Muskels, ein beispielsweise sportmedizinisch signifikanter Teil des Muskels oder ein beispielsweise volumetrisch signifikanter Teil des Muskels, welcher charakteristisch für eine Muskelkontraktion sein kann, in Kontakt mit der Belastungsmesseinrichtung. Ein solcher jeweiliger Teilbereich des Muskels ist in Kontakt mit einem jeweiligen korrelierten "Messpunkt", also einer entsprechenden Messstelle oder einem Messort, welcher an einer jeweiligen "Messpunktposition" innerhalb oder auf der Belastungsmesseinrichtung angeordnet ist. Damit besteht zwischen dem jeweiligen Messpunkt und der dazugehörigen Messpunktposition ein Bezug zum jeweiligen Muskel oder zum jeweiligen Teilbereich des Muskels auf der Belastungsmesseinrichtung, womit ein nachvollziehbarer, geometrisch bestimmter und/oder reproduzierbarer Ort für die jeweilige Belastung detektierbar ist.

Eine "Signaleinheit" ist eine technische, beispielsweise elektrische oder auch elektronische Einheit, welche dazu geeignet ist, an die Person ein entsprechendes Signal in Form eines "Kontraktionssignals", also in Form einer Anweisung zum Durchführen einer Muskelkontraktion, auszugeben. Beispielsweise handelt es sich bei der Signaleinheit um eine Lampe, eine Bildschirmanzeige oder beispielsweise auch ein akustisches Signal.

Mittels der Signaleinheit kann so ein Kontraktionssignal an die Person ausgegeben werden, sodass die Person dann anhand des Kontraktionssignals eine "signalisierte Muskelkontraktion", also eine vorgesehene und durchzuführende Muskelkontraktion durchführt. Ein "erfolgreiches Durchführen" ist beispielsweise dann erreicht, wenn mittels der signalisierten Muskelkontraktion eine ausreichende Belastung auf die Belastungsmesseinrichtung ausgeübt ist.

Dazu wird ein "Vergleich" durchgeführt, welcher ein entsprechendes ermitteltes Belastungsprofil sodann bei einem "Entsprechen" des Belastungsprofils und des Vergleichsbelastungsprofils, also bei einem gemäß dem zu erreichenden Ziel ausreichenden Übereinstimmen des Belastungsprofils mit dem Vergleichsbelastungsprofil, ein "positives Erfolgssignal" ausgibt. Ein solches positives Erfolgssignal ist beispielsweise eine entsprechend zu deutende akustische, optische oder textliche Ausgabe, welche mittels der Steuereinheit initiiert wird. Im Gegensatz dazu beschreibt ein "Nicht-Entsprechen", dass das Belastungsprofil und das Vergleichsbelastungsprofil entsprechend dem gewünschten Trainingserfolg so weit voneinander abweichen, dass der Trainingserfolg nicht mehr sichergestellt ist. In diesem Fall erfolgt dann die Ausgabe eines "negativen Erfolgssignals", welches beispielsweise in Form eines Warntons, einer textlichen Ausgabe zum Wiederholen der entsprechenden signalisierten Muskelkontraktion oder analog dazu ausgegeben wird.

Um die Trainingsvorrichtung besonders anwenderfreundlich auszugestalten, weist die Signaleinheit eine optische Anzeigeeinheit, insbesondere einen Bildschirm, auf, sodass mittels der optischen Anzeigeeinheit ein optisches Kontraktionssignal an die Person ausgebbar ist.

Eine solche "optische Anzeigeeinheit" ist dabei derart ausgestaltet, dass die Person mittels ihrer Augen und damit funktionell unabhängig von entsprechenden körperlichen Bewegungen ein "optisches Kontraktionssignal", also ein mittels der Augen aufnehmbares Signal, mit der Aufforderung zum Durchführen der signalisierten Muskelkontraktion ausgebbar ist.

Erfindungsgemäß weist die Signaleinheit einen mechanischen, insbesondere einen elektromechanischen Aktuator auf, wobei mittels des mechanischen Aktuators ein mechanisches Kontraktionssignal und/oder ein mechanisches Erfolgssignal an die Person ausgebbar ist. Damit kann beispielsweise mittels eines "Aktuators", welcher ein "mechanisches Kontraktionssignal" beispielsweise in Form von Vibrationen, Druckstößen oder einem Einzeldruck ausgeben kann, der Person direkt die Kontraktion eines entsprechenden, beispielsweise auch am Aktuator angeordneten, Muskels durchzuführen. Ein "mechanisches Erfolgssignal" kann dabei beispielsweise in Form eines leichten Vibrierens ausgeführt sein, sodass die Person durch dieses leichte Vibrieren beispielsweise ein erfolgreiches Durchführen der Muskelkontraktion signalisiert bekommt.

Um mittels der Trainingsvorrichtung auch eine aktive Stimulation zu einer Muskelkontraktion durchführen zu können, weist die Signaleinheit einen Elektro-Stimulator auf, wobei mittels des Elektro-Stimulators eine Elektromyostimulation des Muskels ermöglicht ist. Ein "Elektro-Stimulator" besteht beispielsweise aus mehreren am Grundkörper angeordneten Elektroden, welche mit dem entsprechenden Muskel oder der entsprechenden Muskelgruppe in Kontakt sind und ein elektrisches Signal aussenden, mittels dem eine sogenannte "Elektromyostimulation", also ein elektrisches Anregen der Muskelkontraktion von außerhalb des Körpers, durchführbar ist.

In einer Ausführungsform ist der Belastungssensor oder sind die Belastungssensoren ein Drucksensor oder Drucksensoren, wobei der Drucksensor oder die Drucksensoren als schaltender Drucksensor oder schaltende Drucksensoren, als kapazitiver Drucksensor oder kapazitive Drucksensoren und/oder resistiver Drucksensor oder resistive Drucksensoren ausgebildet ist oder sind und/oder mehrere Belastungssensoren insbesondere entlang einer ersten Hauptrichtung und/oder entlang einer zweiten Hauptrichtung in einer matrixartigen Anordnung an der Belastungsmesseinrichtung angeordnet sind.

So kann eine Muskelkontraktion über entsprechende Kräfte oder auch gemessene oder abgeleitete Drücke auf der Belastungsmesseinrichtung detektiert werden. Wird eine Anordnung entlang einer ersten Hauptrichtung sowie entlang einer zweiten Hauptrichtung matrixartig vorgenommen, so kann beispielsweise ein geometrisch definiertes Netzwerk aus Drucksensoren oder anderen Belastungssensoren geschaffen werden, wodurch die Auswertung der Muskelkontraktion an unterschiedlichen Messpunkten stark vereinfacht wird.

Ein "Drucksensor" kann ein Messglied zum Bestimmen eines Drucks, also zum Bestimmen einer Kraft je Fläche, sein wobei ein solcher Drucksensor hier auch in Form eines Kraftsensors ausgeführt sein kann. So kann beispielsweise über die Messpunktposition und eine entsprechende, dem zugehörigen Messpunkt zugeordnete Einflussfläche für den Kraftsensor auf einen Druck, also die gemessene Kraft in Bezug zur jeweiligen Einflussfläche, geschlossen werden. Ein solcher Drucksensor jeder Ausgestaltung kann daher auch synonym als Kraftsensor bezeichnet sein und auch wirken. Ein "schaltender" Drucksensor oder schaltender Kraftsensor kann beispielsweise bei Überschreiten einer Grenzkraft oder eines Grenzdrucks ein Schaltsignal in Form einer 0/1-Information erzeugen, sodass beispielsweise das Belasten des entsprechenden Kraftsensors oder Drucksensors detektiert wird. Wird beispielsweise ein kapazitiver Drucksensor und/oder ein resistiver Drucksensor oder ein anderer geeigneter Sensor eingesetzt, so kann auch qualitativ bestimmt werden, wie groß ein entsprechender Druck oder wie groß eine entsprechende Kraft am jeweiligen Messpunkt ist.

Eine "Hauptrichtung" ist in diesem Zusammenhang beispielsweise eine jeweilige Achse eines Koordinatensystems, wobei hier sowohl kartesische Koordinaten als auch Polarkoordinaten oder eine andere zweckmäßige Anordnung genutzt werden kann, um entsprechende Belastungssensoren matrixartig anzuordnen. Auch sind hier besonders angepasste Schemata oder Anordnungen, beispielsweise auch bogenförmig angepasst an einen Muskelverlauf, zweckdienlich.

Um eine detailliertere Auswertung bezüglich der signalisierten Muskelkontraktion vornehmen zu können, ist die Steuereinheit zum Ermitteln einer Belastungsfläche aus der Messpunktposition mehrerer Messpunkte, einer Belastungsintensität an einem jeweiligen Messpunkt und/oder einem Belastungsvolumen mittels einer Überlagerung, insbesondere mittels einer Multiplikation und/oder mittels einer gewichteten Multiplikation der Belastungsfläche mit der jeweiligen Belastungsintensität am jeweiligen Messpunkt eingerichtet.

Eine "Belastungsfläche" ist beispielsweise eine flächenmäßige Gesamtheit derjenigen Messpunkte, welche beispielsweise eine Belastungsveränderung aufweisen oder welche, sofern das Vergleichsbelastungsprofil beispielsweise einer Ruheposition der Belastungsmesseinrichtung entspricht, demgegenüber eine messbare Belastung, beispielsweise oberhalb eines Grenzwertes zum Unterdrücken von Messungenauigkeiten, aufweisen. Damit ist eine Aussage in Form eines analog zu einer durch Muskelkontraktion wirksamen Kontaktfläche des jeweiligen Muskels mit der Belastungsmesseinrichtung möglich, sodass beispielweise eine Länge, eine Breite oder auch eine Flächengröße, beispielsweise in [cm²], dazu genutzt werden kann, eine Aussage über die Stärke oder auch die Form der Muskelkontraktion zu treffen. Eine "Länge" kann dabei beispielsweise entlang einer Hauptrichtung des Muskels bestimmt sein, eine "Breite" entsprechend orthogonal dazu und eine "Flächengröße" beispielsweise als Flächenintegral, einer Intervallsummation oder einer numerischen Summation der entsprechenden Messpunkte über die flächigen oder auch flächenanalogen räumlichen Dimensionen der Messpunktpositionen, letzteres beispielsweise im Falle einer leicht gewölbten Kontaktfläche zum Muskel. Analog dazu kann eine "Form", also eine Geometrie der Belastungsfläche ermittelt werden, welche beispielsweise eine Aussage über die Muskelkontraktion in bestimmten Teilbereichen des Muskels zulässt, um einen Trainingserfolg zu bewerten.

Eine "Belastungsintensität" beschreibt ein Signal, welches eine Aussage über eine Stärke des jeweiligen Signals geben kann, also beispielsweise zwischen einer Grundbelastung von "0" und einer Maximalbelastung von "1" oder beispielsweise auch in Form einer Kraft in Newton auf einer dem Messzweck angemessenen Skala skaliert ist, sodass eine entsprechende Belastungsintensität an den jeweiligen Messpositionen ein amplitudenbehaftetes Belastungsprofil ergeben.

Um eine noch genauere Aussage über die Gesamtstärke der Muskelkontraktion treffen zu können, wird aus einer Überlagerung der ermittelten Belastungsfläche mit den ermittelten Belastungsintensitäten der Muskelkontraktion an den jeweiligen Messpunkten eine Überlagerungsintensität der Muskelkontraktion ermittelt, wobei die Überlagerung insbesondere mittels einer Multiplikation und/oder einer gewichteten Multiplikation der Belastungsfläche mit der jeweiligen Belastungsintensität am jeweiligen Messpunkt und/oder mit einem Integrieren der Belastungsintensität über der Belastungsfläche, gebildet wird, sodass ein Belastungsvolumen ermittelt ist.

Eine "Überlagerung" der ermittelten Belastungsfläche mit den ermittelten Belastungsintensitäten beschreibt dabei eine mathematische Operation unter der Berücksichtigung mehrerer Dimensionen, beispielsweise der räumlichen Dimensionen der Belastung oder auch einer zeitlichen Dimension der Belastung an bestimmten Messpunkten zu einem bestimmten Zeitpunkt, der entsprechenden Belastung der Muskelkontraktion. So wird beispielsweise eine Multiplikation der Belastungsfläche mit der jeweiligen, insbesondere amplitudenbehafteten Belastungsintensität am jeweiligen Messpunkt durchgeführt oder mathematisch kontinuierlich die Belastungsintensität über der Belastungsfläche in Form einer Integration berechnet oder auch eine gewichtete Multiplikation durchgeführt, in der beispielsweise bestimmte Bereiche, nämlich beispielsweise sportmedizinisch besonders relevante Teilbereiche des Muskels an deren Kontaktfläche mit der Belastungsmesseinrichtung gesondert oder beispielsweise verstärkt berücksichtigt werden. Dabei kann auch ein Integrieren im Sinne eines Bildens eines mathematischen Integrals der Belastungsintensität über der Belastungsfläche derart durchgeführt werden, dass beispielsweise jeweilige Teilintegrale über bestimmte Bereiche der Belastungsfläche gebildet werden oder ein Bestimmen der Überlagerung mittels eines Aufsummierens diskreter Berechnungen an jeweiligen Messpunkten in Form einer numerischen Berechnung als ausreichend genaue Annäherung an das mathematisch genaue Integral durchgeführt werden. Im Ergebnis liegt dann beispielsweise ein aus einer Matrix mit einzelnen Belastungswerten an bestimmten Messpunkten gebildeter, summenartiger Zahlenwert vor, der charakteristisch für die mit dem Muskel durchgeführte Kontraktion ist. Alternativ oder auch ergänzend kann beispielsweise ein Integrieren oder ein Aufsummieren der Messwerte über die Zeit erfolgen, sodass beispielsweise die geleistete Arbeit in Sinne einer Trainingsleistung des jeweiligen Muskels über eine für ein Training gewählte Zeit bestimmt und für die Rückmeldung bezüglich des Trainingserfolges genutz werden kann.

Ein "Belastungsvolumen" ist dabei die Messgröße, welche als dieser Zahlenwert sowohl die Fläche, nämlich die Belastungsfläche, als auch eine entsprechende lokale Belastungsintensität, deren Verteilung oder dergleichen, berücksichtigt und analog zu einer Summation der einzelnen Belastungsintensitäten über der Belastungsfläche abbildet, sodass das Belastungsvolumen kennzeichnend für beispielsweise die mit dem Muskel geleistete Gesamtarbeit zum jeweiligen Messzeitpunkt ist.

In diesem Zusammenhang sei erwähnt, dass das Multiplizieren, das Integrieren oder eine andere mathematische Operation für die vorliegende Erfindung sowohl mathematisch exakt aus entsprechend abgeleiteten Funktionen, als auch numerisch, intervallgeschachtelt oder auf ähnliche Weise durchgeführt werden kann, wobei die jeweilige Durchführungsform dabei beispielsweise ein entsprechendes Raster oder entsprechende Abstände von Messpunkten, in der jeweiligen Anordnung der Messpunktpositionen oder dergleichen, berücksichtigen kann. Erfindungsgemäß ist hierbei nicht ausschließlich die mathematische Präzision des Bestimmens der Belastung am jeweiligen Messpunkt, sondern insbesondere das messtechnische Gesamtbild der entsprechenden Muskelkontraktion auf der Belastungsmesseinrichtung entscheidend, um eine entsprechende Rückmeldung über die Stärke, Ausgestaltung und Qualität der Muskelkontraktion bezüglich eines Trainingserfolges geben zu können.

In einer Ausführungsform weist die Steuereinheit ein Smartphone, einen Tablet-Computer und/oder einen Personal-Computer auf. Mittels solcher, üblicherweise beispielsweise in einem Büro oder in einem Haushalt vorhandener, elektronischer Geräte kann beispielsweise die Steuereinheit auch in Form einer Software auf dem entsprechenden elektronischen Gerät realisiert sein, sodass beispielsweise in einem Büro ein entsprechend ausgerüsteter Stuhl als Trainingsvorrichtung mit einer entsprechenden am Büroarbeitsplatz vorhandenen Personal-Computer verbunden sein kann, sodass die Trainingsvorrichtung durch den vorhandenen Computer und einen speziell ausgestalteten Bürostuhl gebildet wird.

Erfindungsgemäß weist die Trainingsvorrichtung dabei einen Stuhl oder einen Sessel auf. Dabei kann die Trainingsvorrichtung auch im Wesentlichen durch den Stuhl oder den Sessel mit einer entsprechenden zugeordneten Steuereinheit gebildet sein. In einer Ausführungsform ist dabei die Belastungsmesseinrichtung einer Sitzfläche, in einer Rückenlehne, in einer Fußstütze, in einer Armlehne, in einer Kopfstütze und/oder in einer Nackenstütze des Stuhls oder des Sessels und/oder in einer Liegefläche der Liege angeordnet.

Somit können entsprechende Muskeln oder auch Muskelgruppen, welche beispielsweise mit der Rückenlehne, einer Fußstütze oder einer Armlehne, in einer Kopfstütze und/oder in einer Nackenstütze des Stuhls oder des Sessels in Kontakt sind, gezielt trainiert werden.

Weiterhin kann eine nicht-Erfindungsgemäße Trainingsvorrichtung auch eine Matte, eine Sportmatte, eine Yogamatte, ein Kleidungsstück oder dergleichen aufweisen.

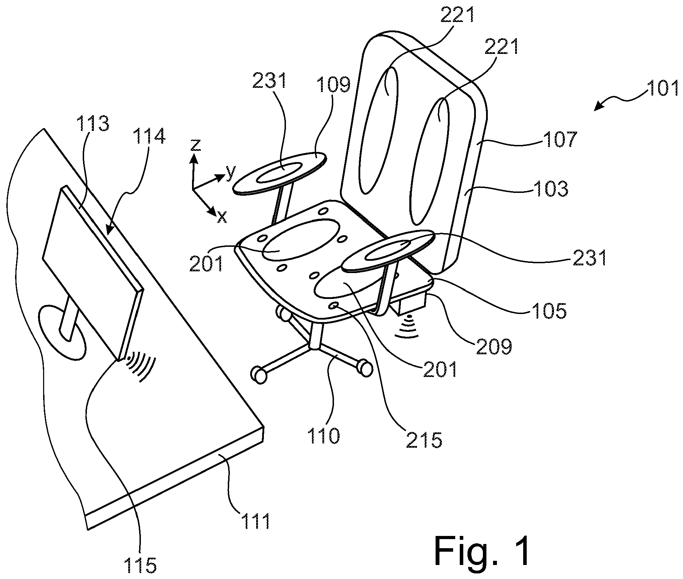

Im Weiteren wird die Erfindung anhand von Ausführungsbeispielen näher erläutert. Es zeigen
- Figur 1: eine schematische Darstellung eines Arbeitsplatzes mit einer integrierten Trainingsvorrichtung in einer isometrischen Ansicht,
- Figur 2: eine schematische Darstellung einer Sensormatte zum Messen von auf die Sensormatte auftretenden Belastungen in einer isometrischen Ansicht, sowie
- Figur 3: ein dreidimensionales Diagramm zum Darstellen entsprechender Sensorsignale von Sensormatten.

Ein Arbeitsplatz 101 weist einen Bürostuhl 103 mit einer Sitzfläche 105, einer Rückenlehne 107 sowie zwei Armlehnen 109 auf. Zudem kann der Bürostuhl eine Kopfstütze und/oder eine Nackenstütze aufweisen (im vorliegenden Beispiel nicht dargestellt). Weiterhin weist der Bürostuhl 103 ein Gestell 110 auf. Der Bürostuhl 103 steht an einem Schreibtisch 111 und weist in seiner Sitzfläche 105 Sensormatten 201, in der Rückenlehne 107 Sensormatten 221 und in den Armlehnen 109 Sensormatten 231 zur Detektion für auf die jeweiligen Bauteile einwirkenden Drücke auf. Weiterhin sind in der Sitzfläche 105 beispielhaft Aktuatoren 215 gezeigt. Auf dem Schreibtisch 111 ist ein Computer 113 mit einem Bildschirm 114 angeordnet, der als üblicher Desktop-PC ausgeführt ist und üblicherweise zum Arbeiten am Arbeitsplatz 101 genutzt wird.

Der Computer 113 weist ein schematisch dargestelltes Kommunikationsmodul 115 auf, welches im gezeigten Beispiel ein Bluetooth-Modul ist. Das Kommunikationsmodul 115 kann damit sowohl Signale aussenden als diese auch empfangen. Der Bürostuhl 103 weist ebenfalls ein Kommunikationsmodul 209 auf, welches mittels des Bluetooth-Protokolls mit dem Kommunikationsmodul 115 Daten austauschen kann.

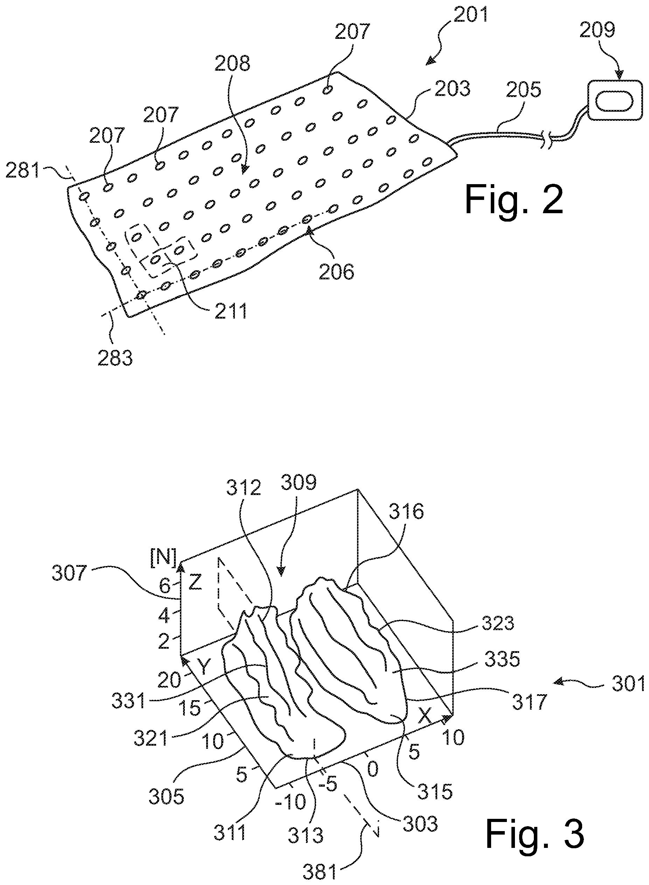

Eine beispielhaft beschriebene Sensormatte 201 mit einer analogen Funktion zu den Sensormatten 221 und Sensormatten 231 weist einen Mattenkörper 203 aus einem Stoff-Gewebe auf. Der Mattenkörper 203 ist mit einem Kabel 205 mit der Kommunikationseinheit 209 verbunden. Innerhalb des Mattenkörpers 203 sind entlang von Querachsen 281 und entlang von Längsachsen 283 mehrere Drucksensoren 207 innerhalb eines Matrixfeldes 208 angeordnet, die technisch als Kraftsensoren ausgeführt sind. Die Korrelation von Kraft zu Druck wird hierbei aus einer gemessenen Kraft an einem jeweiligen Messpunkt 206 und einer jeweiligen Bezugsfläche 211 gebildet, in dem die gemessene Kraft auf die jeweilige Bezugsfläche 211 bezogen wird, sodass ein Druck am jeweiligen Messpunkt 206 angebbar ist. Im Folgenden wird daher nur noch von "Druck" im jeweiligen Zusammenhang gesprochen, auch wenn technisch eine Kraftmessung erfolgt. Die Drucksensoren 207 sind resistive Drucksensoren und können wie oben beschrieben einen orthogonal auf den Mattenkörper 203 einwirkenden lokalen Druck an einem jeweiligen Messpunkt 206 detektieren. Aus der Anordnung im Matrixfeld 208 kann damit für jeden Messpunkt 206 auf eine entsprechende Druckbelastung auf den Mattenkörper 203 geschlossen werden. Hieraus entsteht analog zur jeweiligen Druckbelastung ein jeweiliger Messwert. Das Kommunikationsmodul 209 dient dabei der Aufnahme der von den Drucksensoren 207 durch das Kabel 205 geleiteten Messwerte in Form von Drucksignalen und Weiterleitung an das Kommunikationsmodul 115 des Computers 113, wobei mittels des Computers 113 dann beispielsweise eine Druckkarte (vgl. auch Figur 3) erzeugt werden kann oder auch eine entsprechende tabellenförmige Aufnahme von Sensorsignalen der Drucksensoren 207 über entsprechende Zeitabschnitte oder in Zeitintervallen ermöglicht ist. Im Vorliegenden Beispiel nimmt das Kommunikationsmodul 209 jedes Signal der Drucksensoren 207 in einer Frequenz von 60 Hz, also 60 Messungen pro Sekunde, auf und leitet diese an den Computer 113 weiter.

Die Sensormatte 201 auf jeder Seite der Sitzfläche 105 nimmt entsprechende Druckdaten von einer auf dem Stuhl 103 sitzenden Person auf, und zwar den Druck des jeweils mit der Sensormatte 201 in Kontakt befindlichen Muskels. Dies sei vorliegend beispielhaft der "m. gluteus maximus", also der große Gesäßmuskel sowie der "m.semitendinosus", also einer der hinteren Beugemuskeln des Oberschenkels. In Ruhelage ergibt sich dabei ein bestimmtes Messbild, also eine Druckverteilung aus Eigengewicht der Person, wobei die Person dann in der Folge entsprechende Muskeln für eine Messung anspannt. Beispielhaft ist dazu in einem Diagramm 301 eine entsprechende Funktion 311 und eine Funktion 315 für den jeweiligen Gesäßbereich und jeweiligen Oberschenkel bei Anspannung der Muskeln zu einem bestimmten Zeitpunkt der Messung gezeigt.

Das Diagramm 301 weist eine Abszisse 303 auf, welche eine Richtung quer zur Sitzfläche 105 des Stuhls 103 repräsentiert. Orthogonal dazu weist das Diagramm 301 eine Ordinate 305 auf, welche analog zur Sitztiefe auf der Sitzfläche 105 verläuft. Eine Applikate 307 steht wiederum orthogonal dazu, sodass ein dreidimensionales kartesisches Koordinatensystem mit einem Koordinatenraum 309 aufgespannt ist. Die Abszisse 303 sowie die Ordinate 305 spannen dabei eine Ebene parallel zur Sitzfläche 105 auf, die Applikate 307 stellt eine Achse zur Darstellung eines Drucks auf der Sensormatte 201 dar, und zwar am jeweiligen Messpunkt 206 in der Ebene der Abszisse 303 und der Ordinate 305.

Im Koordinatenraum 309 wird eine Funktion 311 zur Abbildung des Gesäßbereichs und des Oberschenkels der Person auf einer Körperseite sowie eine Funktion 315 zur Abbildung der Muskelgruppen auf der anderen Körperseite dargestellt. Die Funktion 311 weist diverse Messwerte 331 sowie einen Hochpunkt 312, also den Punkt maximalen Drucks, auf, analog dazu weist die Funktion 315 diverse Messwerte 335 sowie einen Hochpunkt 316 auf. Ebenso bilden die Funktion 311 und die Funktion 315 eine Grundfläche 313 sowie eine Grundfläche 317, mit der ein entsprechender Druckbereich auf der Sitzfläche 105 beschrieben ist. Durch die Anspannung der Muskeln, beispielsweise im hinteren Oberschenkelbereich, übt die Person also einen gegenüber der Ruhelage ortsveränderlichen Druck auf die Sitzfläche 105 aus, der mit den Sensormatten 201 detektiert und vom Computer 113 verarbeitet wird. Dabei kann aus dem über der aus der Abszisse 303 und der Ordinate 305 gebildeten x-y-Ebene auch ein jeweiliges Volumen unter der jeweiligen Funktion bestimmt werden, um die geleistete Arbeit des Muskels daraus abzuleiten.

Die Aktuatoren 215, welche analog und wirkungsgleich dazu auch in der Rückenlehne 107 und in den Armlehnen 109 vorhanden sein können, sind als Piezo-Aktuatoren ausgeführt und können, vom Computer 113 über die Kommunikationseinheiten 115 und 209 angesteuert, Vibrationssignale unterschiedlicher Intensität und Frequenz aussenden und damit an eine auf dem Stuhl 103 sitzende Person abgeben.

Der Arbeitsplatz 101 kann mit der beschriebenen Ausrüstung damit als Trainingseinrichtung genutzt werden, beispielsweise wenn durch langes Sitzen am Schreibtisch 111 ein Ermüden entsprechender Muskelgruppen eintritt oder beispielsweise ein entsprechender Zeitablauf mit der Gefahr des Ermüdens eben dieser Muskeln vergangen ist. Auf dem Computer 113 ist eine zum Betreiben der Trainingsvorrichtung dienende Software installiert, welche damit den Computer 113 gleichzeitig zur Steuereinheit für den Bürostuhl 103 ausbildet. Zunächst wird ein entsprechendes Belastungsprofil analog zur Funktion 311 und zur Funktion 315 in Ruhe der Person aufgenommen, wobei dies mittels der Sensormatte 201 in der Sitzfläche 105 geschieht. Gleichzeitig erfolgt mittels der analog ausgebildeten Sensormatte 221 in der Rückenlehne 107 und mittels der analog, jedoch deutlich kleiner ausgebildeten Sensormatten 231 in den Armlehnen 109 ein entsprechendes Aufnehmen der Ruhesignale entsprechender Muskeln.

Am Beispiel der Sitzfläche 105 sei nun eine entsprechende Stimulation und Anregung zur Muskelkontraktion als Training erläutert:

Die Aktuatoren 215 werden, beispielsweise nach Ablauf einer mittels der Sensormatten 201 detektierten bewegungslosen Phase, mittels der Software auf dem Computer 113 über die zwischen dem Kommunikationsmodul 115 und dem Kommunikationsmodul 209 gebildete Übertragungsstrecke angesteuert und in Vibration versetzt, sodass die auf dem Stuhl sitzende Person (nicht dargestellt) die entsprechenden rückseitig am Oberschenkel angeordneten Muskelgruppen angeregt durch die Vibration kontrahieren kann. Sofern mit den Sensormatten 201 eine ausreichende Muskelkontraktion mittels eines Vergleiches der Funktionen 311 und 315 mit entsprechenden Signalen in Ruhelage detektiert oder eine vorgegebene Kontraktionsdauer erreicht ist, wird mittels der Aktuatoren 215 ein leichtes und gegenüber dem zur Muskelkontraktion stimulierenden Signal deutlich geringeres Signal in Form einer leichten Vibration abgegeben, sodass die auf dem Stuhl sitzende Person über den Erfolg oder das Ende der entsprechenden Übung informiert ist. Analog dazu kann auch eine optische Anzeige auf dem Bildschirm 114 und/oder eine akustische Anzeige erfolgen. Sofern die entsprechende Muskelkontraktion nicht zufriedenstellend durchgeführt ist, kann beispielsweise die Stimulation mittels der Aktuatoren 215 wiederholt werden, sodass die Muskelkontraktion dann durchgeführt wird. Alternativ oder auch ergänzend kann, beispielsweise in der Sitzfläche 105, auch ein Elektro-Stimulator (nicht dargestellt) angeordnet sein, der mittels elektrischer Impulse eine Elektromyostimulation durchführt und damit die Muskelkontraktion unterstützt oder auslöst.

Analog dazu würde beispielsweise mittels der Sensormatten 221 und entsprechenden Aktuatoren (nicht dargestellt) in der Rückenlehne 107 die Stimulation der Rückenmuskulatur durch beispielsweise eine initiierte Rückenbewegung oder analog dazu die Stimulation der Unterarmmuskulatur in Zusammenhang mit den Sensormatten 231 in den Armlehnen 109 und entsprechenden Aktuatoren (nicht dargestellt) einer Anzeige auf dem Bildschirm 114 oder einem gleichwertigen Signal initiiert.

### Bezugszeichenliste

- 101: Arbeitsplatz
- 103: Bürostuhl
- 105: Sitzfläche
- 107: Rückenlehne
- 109: Armlehne
- 110: Gestell
- 111: Schreibtisch
- 113: Computer
- 114: Bildschirm
- 115: Kommunikationsmodul
- 201: Sensormatte
- 203: Mattenkörper
- 205: Kabel
- 206: Messpunkt
- 207: Drucksensor
- 208: Matrixfeld
- 209: Kommunikationsmodul
- 211: Bezugsfläche
- 215: Aktuator
- 221: Sensormatte
- 231: Sensormatte
- 281: Querachse
- 283: Längsachse
- 301: Diagramm
- 303: Abszisse
- 305: Ordinate
- 307: Applikate
- 309: Koordinatenraum
- 311: Funktion
- 312: Hochpunkt
- 313: Grundfläche
- 314: Referenzfläche
- 315: Funktion
- 316: Hochpunkt
- 317: Grundfläche
- 318: Referenzfläche
- 321: Druckvolumen
- 323: Druckvolumen
- 331: Messwert
- 335: Messwert
- 381: Schnittebene

## Patentansprüche

1. Trainingsvorrichtung (101), zum Durchführen eines Muskeltrainings für einen Muskel, insbesondere für einen bestimmten Muskel, einer Person mit einer Steuereinheit (113), einem Grundkörper (103) und einer am Grundkörper (103) angeordneten Belastungsmesseinrichtung (201, 221, 231), wobei der Muskel, insbesondere der bestimmte Muskel, in einer Nutzungsposition der Person in Kontakt mit der Belastungsmesseinrichtung (201, 221, 231) ist und die Belastungsmesseinrichtung (201, 221, 231) einen Belastungssensor (207) oder mehrere Belastungssensoren (207) zum Bestimmen einer durch die Person auf die Belastungsmesseinrichtung (201, 221, 231) ausgeübten Belastung an einer jeweiligen mit einem Teilbereich des Muskels korrelierten Messpunkt (206) an einer jeweiligen Messpunktposition aufweist, wobei die Steuereinheit (113) eine Signaleinheit (114, 215) aufweist und die Steuereinheit (113) derart eingerichtet ist, dass mittels der Signaleinheit (114, 215) ein Kontraktionssignal an die Person ausgebbar ist, sodass die Person anhand des Kontraktionssignals eine signalisierte Muskelkontraktion durchführt und ein erfolgreiches Durchführen der signalisierten Muskelkontraktion mittels eines Vergleichs eines mittels der Belastungsmesseinrichtung (201, 221, 231) ermittelten Belastungsprofils (311, 315) mit einem Vergleichsbelastungsprofil überprüfbar ist, indem bei einem Entsprechen des Belastungsprofils (311, 315) und des Vergleichsbelastungsprofils ein positives Erfolgssignal und bei einem Nicht-Entsprechen des Belastungsprofils (311, 315) und des Vergleichsbelastungsprofils ein negatives Erfolgssignal ausgegeben wird, **dadurch gekennzeichnet, dass** die Trainingsvorrichtung einen Stuhl oder einen Sessel aufweist, und dass die Signaleinheit (114, 215) einen mechanischen Aktuator (215) aufweist, wobei mittels des mechanischen Aktuators (215) ein mechanisches Kontraktionssignal und/oder ein mechanisches Erfolgssignal an die Person ausgebbar ist.

2. Trainingsvorrichtung gemäß Anspruch 1, **dadurch gekennzeichnet, dass** die Signaleinheit (114, 215) eine optische Anzeigeeinheit (114), insbesondere einen Bildschirm (114), aufweist, sodass mittels der optischen Anzeigeeinheit (114) ein optisches Kontraktionssignal an die Person ausgebbar ist.

3. Trainingsvorrichtung gemäß Anspruch 1 oder 2, **dadurch gekennzeichnet, dass** die Signaleinheit (114, 215) einen elektromechanischen Aktuator (215) aufweist, wobei mittels des elektromechanischen Aktuators (215) ein mechanisches Kontraktionssignal und/oder ein mechanisches Erfolgssignal an die Person ausgebbar ist.

4. Trainingsvorrichtung gemäß einem der vorherigen Ansprüche, **dadurch gekennzeichnet, dass** die Signaleinheit (114, 215) einen Elektro-Stimulator aufweist, wobei mittels des Elektro-Stimulators eine Elektromyostimulation des Muskels ermöglicht ist.

5. Trainingsvorrichtung gemäß einem der vorherigen Ansprüche, **dadurch gekennzeichnet, dass** der Belastungssensor (207) oder die Belastungssensoren (207) ein Drucksensor (207) oder Drucksensoren (207) ist oder sind, wobei der Drucksensor (207) oder die Drucksensoren (207) als schaltender Drucksensor oder schaltende Drucksensoren, als kapazitiver Drucksensor oder kapazitive Drucksensoren und/oder als resistiver Drucksensor oder resistive Drucksensoren ausgebildet ist oder sind und/oder mehrere Belastungssensoren (207) insbesondere entlang einer ersten Hauptrichtung (281) und/oder entlang einer zweiten Hauptrichtung (283) in einer matrixartigen Anordnung (208) an der Belastungsmesseinrichtung (201, 221, 231) angeordnet sind.

6. Trainingsvorrichtung gemäß einem der vorherigen Ansprüche, **dadurch gekennzeichnet, dass** die Steuereinheit (113) zum Ermitteln einer Belastungsfläche (313, 317) aus der Messpunktposition (206) mehrerer Messpunkte, einer Belastungsintensität (312, 316) an einem jeweiligen Messpunkt und/oder einem Belastungsvolumen mittels einer Überlagerung, insbesondere mittels einer Multiplikation und/oder einer gewichteten Multiplikation der Belastungsfläche (313, 317) mit der jeweiligen Belastungsintensität (312, 316) am jeweiligen Messpunkt, eingerichtet ist.

7. Trainingsvorrichtung gemäß einem der vorherigen Ansprüche, **dadurch gekennzeichnet, dass** die Steuereinheit (113) ein Smartphone, einen Tablet-Computer und/oder einen Personal-Computer (113) aufweist.

8. Trainingsvorrichtung gemäß einem der vorherigen Ansprüche, **dadurch gekennzeichnet, dass** die Belastungsmesseinrichtung (201, 221, 231) in einer Sitzfläche (105), in einer Rückenlehne (107), in einer Fußstütze, in einer Armlehne (109), in einer Kopfstütze und/oder in einer Nackenstütze des Stuhls (103) oder des Sessels angeordnet ist.

## Claims

1. Training device (101), for carrying out muscle training for a muscle, in particular for a specific muscle, of a person, comprising a control unit (113), a main body (103) and a load measuring device (201, 221, 231) arranged on the main body (103), wherein the muscle, in particular the specific muscle, is in contact with the load measuring device (201, 221, 231) in a use position of the person and the load measuring device (201, 221, 231) has a load sensor (207) or a plurality of load sensors (207) for determining a load exerted by the person onto the load measuring device (201, 221, 231) at a respective measurement point (206) correlated with a region of the muscle at a respective measurement point position,
wherein the control unit (113) has a signal unit (114, 215) and the control unit (113) is set up in such a way that a contraction signal can be emitted to the person by the signal unit (114, 215) so that the person performs a signalled muscle contraction on the basis of the contraction signal and successful execution of the signalled muscle contraction can be verified by comparing a load profile (311, 315) determined by the load measuring device (201, 221, 231) with a comparison load profile, in that a positive success signal is emitted in the event that the load profile (311, 315) and the comparison load profile correspond and a negative success signal is emitted in the event that the load profile (311, 315) and the comparison load profile do not correspond, **characterised in that** the training device has a chair or an armchair, and **in that**
the signal unit (114, 215) has a mechanical actuator (215), wherein a mechanical contraction signal and/or a mechanical success signal can be emitted to the person by the mechanical actuator (215).

2. Training device according to claim 1, **characterised in that** the signal unit (114, 215) has an optical display unit (114), in particular a screen (114), so that an optical contraction signal can be output to the person by the optical display unit (114).

3. Training device according to claim 1 or 2, **characterised in that** the signal unit (114, 215) has an electromechanical actuator (215), wherein a mechanical contraction signal and/or a mechanical success signal can be emitted to the person by the electromechanical actuator (215).

4. Training device according to any one of the preceding claims, **characterised in that** the signal unit (114, 215) has an electro-stimulator, wherein an electromyostimulation of the muscle is made possible by the electro-stimulator.

5. Training device according to any one of the preceding claims, **characterised in that** the load sensor (207) or the load sensors (207) is or are a pressure sensor (207) or pressure sensors (207), wherein the pressure sensor (207) or the pressure sensors (207) is or are formed as switching pressure sensor or switching pressure sensors, as capacitive pressure sensor or capacitive pressure sensors and/or as resistive pressure sensor or resistive pressure sensors and/or a plurality of load sensors (207) are arranged on the load measurement device (201, 221, 231) in particular in a first main direction (281) and/or in a second main direction (283) in a matrix-like arrangement (208).

6. Training device according to any one of the preceding claims, **characterised in that** the control unit (113) is set up to determine a load area (313, 317) from the measurement point position (206) of a plurality of measurement points, a load intensity (312, 316) at a respective measurement point and/or a load volume by a superimposition, in particular by means of a multiplication and/or a weighted multiplication, of the load area (313, 317) with the respective load intensity (312, 316) at the respective measurement point.

7. Training device according to any one of the preceding claims, **characterised in that** the control unit (113) has a smartphone, a tablet computer and/or a personal computer (113).

8. Training device according to any one of the preceding claims, **characterised in that** the load measurement device (201, 221, 231) is arranged in a seat surface (105), in a backrest (107), in a footrest, in an armrest (109), in a headrest and/or in a neck support of the chair (103) or the armchair.

## Revendications

1. Dispositif d'entraînement (101) pour effectuer un entraînement musculaire pour un muscle, notamment pour un muscle déterminé, d'une personne avec une unité de commande (113), un corps de base (103) et un appareil de mesure de charge (201, 221, 231) agencé sur le corps de base (103), le muscle, notamment le muscle déterminé, étant en contact avec l'appareil de mesure de charge (201, 221, 231) dans une position d'utilisation de la personne, et l'appareil de mesure de charge (201, 221, 231) comprenant un capteur de charge (207) ou plusieurs capteurs de charge (207) pour déterminer une charge exercée par la personne sur l'appareil de mesure de charge (201, 221, 231) en un point de mesure respectif (206) corrélé à une zone partielle du muscle en une position de point de mesure respective,
l'unité de commande (113) présentant une unité de signalisation (114, 215) et l'unité de commande (113) étant adaptée de telle sorte qu'un signal de contraction peut être délivré à la personne au moyen de l'unité de signalisation (114, 215), de telle sorte que la personne effectue une contraction musculaire signalée à l'aide du signal de contraction et une exécution réussie de la contraction musculaire signalée peut être vérifiée au moyen d'une comparaison d'un profil de charge (311, 315) déterminé au moyen de l'appareil de mesure de charge (201, 221, 231) avec un profil de charge de comparaison, en émettant un signal de réussite positif lors d'une correspondance du profil de charge (311, 315) et du profil de charge de comparaison et un signal de réussite négatif lors d'une non-correspondance du profil de charge (311, 315) et du profil de charge de comparaison, **caractérisé en ce que** le dispositif d'entraînement présente une chaise ou un fauteuil, et **en ce que**
l'unité de signalisation (114, 215) présente un actionneur mécanique (215), un signal de contraction mécanique et/ou un signal de réussite mécanique pouvant être délivré à la personne au moyen de l'actionneur mécanique (215).

2. Dispositif d'entraînement selon la revendication 1, **caractérisé en ce que** l'unité de signalisation (114, 215) présente une unité d'affichage optique (114), notamment un écran (114), de telle sorte qu'un signal de contraction optique peut être délivré à la personne au moyen de l'unité d'affichage optique (114).

3. Dispositif d'entraînement selon la revendication 1 ou 2, **caractérisé en ce que** l'unité de signalisation (114, 215) présente un actionneur électromécanique (215), un signal de contraction mécanique et/ou un signal de réussite mécanique pouvant être délivré à la personne au moyen de l'actionneur électromécanique (215).

4. Dispositif d'entraînement selon l'une quelconque des revendications précédentes, **caractérisé en ce que** l'unité de signalisation (114, 215) présente un électro-stimulateur, l'électro-stimulateur permettant une électromyostimulation du muscle.

5. Dispositif d'entraînement selon l'une quelconque des revendications précédentes, **caractérisé en ce que** le capteur de charge (207) ou les capteurs de charge (207) est ou sont un capteur de pression (207) ou des capteurs de pression (207), le capteur de pression (207) ou les capteurs de pression (207) étant réalisés sous forme de capteur de pression à commutation ou de capteurs de pression à commutation, sous forme de capteur de pression capacitif ou de capteurs de pression capacitifs et/ou sous forme de capteur de pression résistif ou de capteurs de pression résistifs et/ou plusieurs capteurs de charge (207) étant agencés notamment le long d'une première direction principale (281) et/ou le long d'une deuxième direction principale (283) dans un agencement de type matrice (208) sur l'appareil de mesure de charge (201, 221, 231).

6. Dispositif d'entraînement selon l'une quelconque des revendications précédentes, **caractérisé en ce que** l'unité de commande (113) est adaptée pour déterminer une surface de charge (313, 317) à partir de la position de point de mesure (206) de plusieurs points de mesure, une intensité de charge (312, 316) à un point de mesure respectif et/ou un volume de charge au moyen d'une superposition, notamment au moyen d'une multiplication et/ou d'une multiplication pondérée de la surface de charge (313, 317) par l'intensité de charge respective (312, 316) au point de mesure respectif,

7. Dispositif d'entraînement selon l'une quelconque des revendications précédentes, **caractérisé en ce que** l'unité de commande (113) présente un smartphone, une tablette et/ou un ordinateur personnel (113).

8. Dispositif d'entraînement selon l'une quelconque des revendications précédentes, **caractérisé en ce que** l'appareil de mesure de charge (201, 221, 231) est agencé dans une surface d'assise (105), dans un dossier (107), dans un repose-pieds, dans un accoudoir (109), dans un appui-tête et/ou dans un appui-nuque de la chaise (103) ou du fauteuil.
